(19) Europäisches Patentamt
European Patent Office
Office européen des brevets

(11) EP 0 873 109 B1

(12) EUROPÄISCHE PATENTSCHRIFT

(45) Veröffentlichungstag und Bekanntmachung des Hinweises auf die Patenterteilung:
**04.02.2004 Patentblatt 2004/06**

(21) Anmeldenummer: **97943909.8**

(22) Anmeldetag: **27.09.1997**

(51) Int Cl.7: **A61K 7/13**

(86) Internationale Anmeldenummer:
**PCT/EP1997/005305**

(87) Internationale Veröffentlichungsnummer:
**WO 1998/020847 (22.05.1998 Gazette 1998/20)**

(54) **FÄRBEMITTEL ZUR ERZEUGUNG VON METAMERIE-EFFEKTEN AUF KERATINFASERN**

COLORANT FOR PRODUCING METAMERIC EFFECTS ON KERATIN FIBRES

COLORANT POUR PRODUIRE DES EFFETS METAMERES SUR DES FIBRES KERATINIQUES

(84) Benannte Vertragsstaaten:
**DE ES FR GB IT**

(30) Priorität: **12.11.1996 DE 19646609**

(43) Veröffentlichungstag der Anmeldung:
**28.10.1998 Patentblatt 1998/44**

(73) Patentinhaber: **Wella Aktiengesellschaft**
**64274 Darmstadt (DE)**

(72) Erfinder:
- **GÖTTEL, Otto**
**CH-1723 Marly (CH)**
- **BRAUN, Hans-Jürgen**
**CH-3182 Überstorf (CH)**

(56) Entgegenhaltungen:
**EP-A- 0 728 464**
**DE-A- 1 940 085**
**DE-A- 4 422 603**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 873 109 B1

Printed by Jouve, 75001 PARIS (FR)

## Beschreibung

Die vorliegende Erfindung betrifft eine Zusammensetzung von Farbstoff-Vorstufen, die im oxidativen System System Färbungen von Keratinfasern, insbesondere menschlichen Haaren, erzeugt, die bei diffusem Licht einen natürlichen Farbton, bei hellem Licht oder direkter Sonne dagegen deutliche rote bis blaue Reflexe ergeben (sogenannter Metamerie-Effekt).

Bekannt sind derartige Farbeffekte vor allem bei den Völkern im östlichen asiatischen Raum, wo die die Haarfarbe von Natur aus schwarz ist und im Sonnenlicht blaue Reflexe aufweist.

Auf dem Gebiet der Haarfärbung haben Oxidationsfarbstoffe eine wesentliche Bedeutung erlangt. Die Färbung entsteht hierbei durch Reaktion bestimmter Entwickler- und Kupplersubstanzen in Gegenwart eines Oxidationsmittels. Von besonderer Bedeutung sind hierbei Haarfärbemittel zur Färbung im Naturtonbereich. Daneben lassen sich durch Kombination geeigneter Oxidationsfarbstoff-Vorstufen auch zeitgemäße modische Farbnuancen erzeugen.

Aufgrund von neuen Modetrends werden in verstärktem Maße abgewandelte Naturtöne, dies sind beispielsweise Brauntöne mit ausgeprägten Kupfer- oder Aubergine-Nuancierungen, gewünscht.

Neben der Erzeugung von Farbeffekten werden an Oxidationsfarbstoffe, die zur Behandlung menschlicher Haare vorgesehen sind, eine Vielzahl von zusätzlichen Anforderungen gestellt. So sollen die enthaltenen Farbstoffe in toxikologischer und dermatologischer Hinsicht unbedenklich sowie nicht sensibilisierend sein. Ferner wird für die erzielten Haarfärbungen eine gute Licht-, Schweiß-, Dauerwell-, Säure-, Basen- und Reibeechtheit gefordert. In jedem Fall müssen solche Haarfärbungen bei normaler, das heißt nicht übermäßiger, Einwirkung von Licht, Reibung oder chemischen Mitteln über einen Zeitraum von mindestens vier bis sechs Wochen stabil bleiben. Außerdem ist es erforderlich, daß durch Kombination geeigneter Entwickler- und Kupplerkomponenten eine breite Palette verschiedener Farbnuancen erzeugt werden kann.

Ein weiteres Problem besteht in der Praxis in dem in Abhängigkeit von der Haarbeschaffenheit unterschiedlichen Aufziehverhalten der Farbstoffe, wodurch eine ungleichmäßige Anfärbung der Haare erfolgt. Normalerweise neigen die verwendeten Farbstoffe dazu, auf geschädigten Haarpartien stärker aufzuziehen als auf ungeschädigten. Aus diesem Grund werden in der Regel die durch übliche Alterungsprozesse und Umwelteinflüsse (zum Beispiel Sonnenlicht, Haarwäschen, Färbe- und Dauerwellbehandlungen) stärker geschädigten Haarspitzen intensiver gefärbt als der in der Regel ungeschädigte Haaransatz und die weniger geschädigten Haarpartien. Hierdurch kann ein unnatürliches, ungleichmäßiges und völlig unbefriedigendes Färbeergebnis bewirkt werden.

Es bestand daher die Aufgabe, Haarfärbemittel zur Verfügung zu stellen, welche die vorgenannten Nachteile nicht aufweisen und gleichzeitig eine Vielzahl von Farbreflexen ermöglichen.

Es wurde nun überraschenderweise gefunden, daß bei Verwendung einer Haarfarbträgermasse gemäß der vorliegenden Erfindung, welche eine Kombination aus bestimmten 4,5-Diaminopyrazolen und bestimmten Benzoxazinen enthält, die vorstehend beschriebene Aufgabenstellung in hervorragender Weise gelöst wird.

Die erfindungsgemäße Haarfarbträgermasse ermöglicht eine gleichmäßige und intensive Färbung von unterschiedlich vorgeschädigtem Haar, wobei das Haar Farbreflexe erhält, welche bei diffusem Licht kaum wahrnehmbar sind, während sie bei direkter Sonneneinstrahlung in verschiedenen Farbtönen mit teilweise metallischem Glanz erscheinen. Daneben zeichnen sich die erhaltenen Färbungen dadurch aus, daß sie bei diffusem Licht oder Kunstlicht in einem unauffälligen, naturnahen Ton erscheinen, während im direkten Sonnenlicht die genannten Farbreflexe auftreten, wobei nicht nur blaue Reflexe sondern auch eine Vielzahl weiterer Farbreflexe im hellroten bis blauvioletten Bereich erzeugt werden kann.

Gegenstand der vorliegenden Anmeldung ist daher eine Haarfarbträgermasse, welche dadurch gekennzeichnet ist, daß sie als Entwicklersubstanz mindestens ein Diaminopyrazol der allgemeinen Formel (1) und als Kupplersubstanz mindestens ein Benzoxazin der allgemeinen Formel (II) enthält,

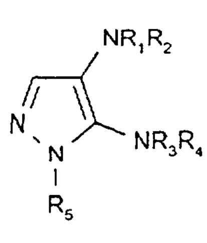

(I)

;

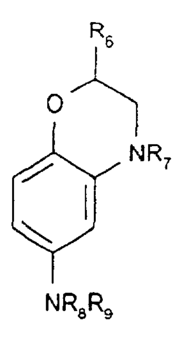

(II)

wobei in der allgemeinen Formel (I) die Substituenten R1 bis R5 unabgängig voneinander Wasserstoff, eine geradkettige oder verzweigte Alkylgruppe mit 1 bis 6 Kohlenstoffatomen, eine geradkettige oder verzweigte Hydroxyalkylgruppe mit 2 bis 4 Kohlenstoffatomen, eine geradkettige oder verzweigte Poly(hydroxy)alkylgruppe mit 3 bis 4 Kohlenstoffatomen oder einen unsubstituierten oder substituierten Benzylrest bedeuten können; R1 und R2 sowie R3 und R4 jeweils ein heterocyclisches System bilden können; R1 oder R2 mit R3 oder R4 verbrückt sein kann; R5 einen substituierten oder unsubstituierten Phenylrest bedeuten kann und R5 mit R3 oder R4 verbrückt sein kann, während in der allgemeinen Formel (II) R6 gleich Wasserstoff oder einer verzweigten oder unverzweigten Alkylgruppe mit 1 bis 14 Kohlenstoffatomen; R7 gleich Wasserstoff, einer geradkettigen oder verzweigten Alkylgruppe mit 1 bis 6 Kohlenstoffatomen, einer geradkettigen oder verzweigten Hydroxyalkylgruppe mit 2 bis 4 Kohlenstoffatomen oder einem Benzylrest sein kann; und R8 und R9 unabhängig voneinander gleich einer geradkettigen oder verzweigten Alkylgruppe mit 1 bis 6 Kohlenstoffatomen, einer geradkettigen oder verzweigten Hydroxyalkylgruppe mit 2 bis 4 Kohlenstoffatomen oder einer geradkettigen oder verzweigten Poly(hydroxy)alkylgruppe mit 3 bis 4 Kohlenstoffatomen sein kann.

Vorzugsweise bedeuten in der Formel (I) R1und R2 Wasserstoff; R3 und R4 Wasserstoff, eine Methylgruppe oder eine Hydroxyethylgruppe bedeuten und R5 eine Methylgruppe, eine Ethylgruppe, eine Isopropylgruppe, eine Hydroxyethylgruppe oder eine Benzylgruppe.

In der allgemeinen Formel (II) haben die Restgruppen vorzugsweise die folgende Bedeutung: R6 gleich Wasserstoff oder einer Methylgruppe und R7, R8 und R9 gleich Wasserstoff.

Zur Erzeugung von speziellen Farbeffekten können dem Haarfärbemittel weitere Oxidationsfarbstoff-Vorstufen zugesetzt werden, beispielsweise Entwicklersubstanzen aus den Klassen der p-Phenylendiamine und p-Aminophenole, insbesondere 2,5-Diaminotoluol, 2-(2',5'-Diaminophenyl)-ethanol, 2-(2',5'-Diaminophenoxy)ethanol, 4-Aminophenol, 4-Amino-2-aminomethylphenol, 4-Amino-2-[(2'-hydroxyethyl)-aminomethyl]phenol, 4-Amino-3-methylphenol beziehungsweise deren Addukte mit organischen oder anorganischen Säuren, und Kupplersubstanzen aus den Klassen der m-Phenylendiamine, m-Aminophenole und Resorcine, insbesondere 2,4-Diamino-phenoxyethanol, 2,4-Diamino-5-fluortoluol, 2-Amino-4-(2'-hydroxyethyl)-aminoanisol, 2-Amino-4-chlor-6-methylphenol, 5-Amino-2-methylphenol, 4-Amino-2-hydroxyphenoxy-ethanol, 4-Amino-5-fluor-2-hydroxytoluol, 4-Amino-5-ethoxy-2-hydroxytoluol, Resorcin, 4-Chlorresorcin, 4,6-Dichlor-resorcin, 2-Methylresorcin, 4-Hydroxy-1,2-methylendioxybenzol, 4-(2'-Hydroxyethyl)amino-1,2-methylendioxybenzol, 4-Hydroxyindol, das Pyridinderivat 3,5-Diamino-2,6-dimethoxypyridin beziehungsweise deren Addukte mit organischen oder anorganischen Säuren sowie 1-Naphthol.

Zur Optimierung des Farbergebnisses können dem Haarfärbemittel weiterhin Verbindungen aus der Gruppe der direktziehenden Farbstoffe, beispielsweise 2-Amino-4,6-dinitrophenol, 2-Ethylamino-4,6-dinitrophenol, 2-[(2'-Hydroxyethyl)amino]-4,6-dinitrophenol oder Farbstoffe der allgemeinen Formel (III)

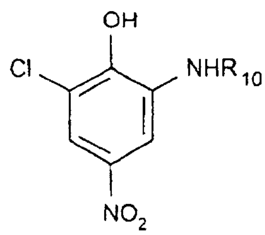

(III)

,

worin R10 Wasserstoff, eine Methylgruppe, eine Ethylgruppe oder eine Hydroxyethylgruppe bedeutet, zugesetzt werden.

Weitere geeignete direkt auf das Haar aufziehende Farbstoffe werden in "Hair Dyes" von J. C. Johnson, Noyes Data Corp., Park Ridge, USA (1973) auf den Seiten 3 bis 91 und 113 bis 139 beschrieben (ISBN: 0-8155-0477-2).

Die erfindungsgemäße Farbträgermasse enthält die vorstehend beschriebenen erfindungsgemäßen Kombinationen von oxidativen Haarfarbstoffen und gegebenenfalls direktziehenden Farbstoffen als solche oder in Form von physiologisch verträglichen Salzen, beispielsweise Hydrochloride, Hydrobromide, Sulfate oder Tartrate oder im Fall von Phenolen als Alkaliphenolate.

Die Gesamtkonzentration an Farbvorstufen beträgt 0,1 bis 10 Gewichtsprozent, bevorzugt 0,2 bis 5 Gewichtsprozent. Die Konzentration der einzelnen Haarfarbstoffe beträgt 0,01 bis 5 Gewichtsprozent, vorzugsweise 0,1 bis 4 Gewichtsprozent.

Darüberhinaus können in der Farbträgermasse noch übliche kosmetische Zusätze, beispielsweise Antioxidantien wie Ascorbinsäure, Thioglykolsäure oder Natriumsulfit; Parfümöle; Komplexbildner; Netzmittel; Emulgatoren; Verdicker; Puffersysteme; Konservierungsstoffe; Pflegestoffe und andere vorhanden sein.

Die vorstehend beschriebene erfindungsgemäße Kombination von oxidativen Haarfarbvorstufen und gegebenenfalls direktziehenden Farbstoffen wird zur Färbung in einem geeigneten kosmetischen Träger appliziert und

ermöglicht eine gleichmäßige Färbung vom Haaransatz bis zur Haarspitze im leicht abgewandelten Naturtonbereich, insbesondere aber Färbungen im Naturtonbereich mit modischen Reflexen.

Die vorzüglichen Eigenschaften der neuen Farbstoffkombinationen zeigen sich insbesondere auf durch Licht und Wetter geschädigtem oder auf dauergewellten Haaren. Die Oxidationsfarbstoffe verfügen dabei über eine sehr gute Auswaschbeständigkeit sowie eine hervorragende Lichtstabilität.

Der Gegenstand der vorliegenden Anmeldung betrifft daher auch ein Mittel zur oxidativen Färbung von Haaren, welches durch Vermischen der erfindungsgemäßen Farbträgermasse mit einem Oxidationsmittel unmittelbar vor der Anwendung hergestellt wird.

Die Zubereitungsform für die Farbträgermasse sowie für das gebrauchsfertige Oxidationshaarfärbemittel kann beispielsweise eine Lösung, insbesondere eine wäßrige oder wäßrig-alkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, n-Propanol und Isopropanol oder Glykole wie Glycerin und 1,2-Propylenglykol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent (bezogen auf die Farbträgermasse), die Verdicker in einer Menge von etwa 0,1 bis 25 Gewichtsprozent (bezogen auf die Farbträgermasse) und die Pflegestoffe in einer Konzentration von etwa 0,1 bis 5,0 Gewichtsprozent (bezogen auf die Farbträgermasse).

Das gebrauchsfertige erfindungsgemäße Haarfärbemittel wird durch Mischen der erfindungsgemäßen Farbträgermasse mit einem flüssigen Oxidationsmittel unmittelbar vor der Anwendung hergestellt.

Die Farbträgermasse und das Oxidationsmittel werden hierbei im Gewichtsverhältnis von etwa 5:1 bis 1:3 miteinander vermischt, wobei ein Gewichtsverhältnis von etwa 1:1 bis 1:2 besonders bevorzugt ist.

Der pH-Wert des gebrauchsfertigen erfindungsgemäßen Haarfärbemittel stellt sich bei der Mischung der vorzugsweise alkalisch eingestellten Farbträgermasse mit dem meist sauer eingestellten Oxidationsmittel auf einen pH-Wert ein, der durch die Alkalimengen in der Farbträgermasse und die Säuremengen im Oxidationsmittel sowie durch das Mischungsverhältnis beinflußt wird. Der pH-Wert des fertigen Haarfärbemittels liegt bei etwa 3 bis 11, vorzugsweise etwa 5 bis 9.

Für die Einstellung des jeweiligen pH-Wertes der Farbträgermasse und des Oxidationsmittels können je nach dem gewünschten pH-Wert organische und anorganische Säuren, wie zum Beispiel Phosphorsäure, Ascorbinsäure und Milchsäure, oder Alkalien wie zum Beispiel Monoethanolamin, Triethanolamin, 2-Amino-2-methyl-1-propanol, Ammoniak, Natronlauge, Kalilauge oder Tris-(hydroxymethyl)-aminomethan, verwendet werden.

Für die Anwendung zur oxidativen Färbung von Haaren vermischt man die vorstehend beschriebene Farbträgermasse unmittelbar vor dem Gebrauch mit einem Oxidationsmittel und trägt eine für die Haarfärbebehandlung ausreichende Menge, je nach Haarfülle im allgemeinen etwa 60 bis 200 Gramm des erhaltenen gebrauchsfertigen Oxidationshaarfärbemittels auf das Haar auf.

Als Oxidationsmittel kommen hauptsächlich Wasserstoffperoxid oder dessen Additionsverbindungen an Harnstoff, Melamin oder Natriumbromat in Form einer 1 bis 12prozentigen, vorzugsweise 6prozentigen, wäßrigen Lösung in Betracht, wobei Wasserstoffperoxid besonders bevorzugt wird.

Man läßt das erfindungsgemäße Haarfärbemittel bei 15 bis 50 Grad Celsius etwa 10 bis 45 Minuten lang, vorzugsweise 30 Minuten lang, auf das Haar einwirken, spült sodann das Haar mit Wasser und trocknet es. Gegebenenfalls wird im Anschluss an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die nachfolgenden Beispiele sollen den Gegenstand näher erläutern, ohne ihn auf diese Beispiele zu beschränken:

**Beispiele**

**Beispiele 1 bis 6:** Haarfärbelösungen mit einem basischen pH-Wert

Es wird folgende Färbelösung hergestellt:

| | |
|---|---|
| 10,0 g | Isopropanol |
| 10,0 g | Natrium-laurylalkohol-diglykolethersulfat (28prozentige wäßrige Lösung) |
| 10,0 g | Ammoniak (25prozentige wäßrige Lösung) |
| 0,3 g | Ascorbinsäure |
| X g | Farbvorstufen gemäß Tabelle 1 |
| ad 100 g | Wasser, vollentsalzt |

Zur Anwendung werden 10 g Haarfärbelösung mit 10 g Wasserstoffperoxid-Lösung (6prozentige wäßrige Lösung) gemischt. Das erhaltene gebrauchsfertige Oxidationshaarfärbemittel wird auf das Haar aufgetragen. Nach einer Einwirkungszeit von 30 Minuten bei 40 °C werden die Haare mit Wasser gespült, shampooniert und getrocknet.

Die erhaltenen Färbungen sind in der nachfolgenden Tabelle 1 zusammengefaßt.

Tabelle 1

| **Beispiel** | **1** | **2** | **3** | **4** | **5** | **6** |
|---|---|---|---|---|---|---|
| **Farbvorstufe** | | | | | | |
| 4,5-Diamino-1-(2'-hydroxyethyl)-1H-pyrazol-sulfat | 1,4 g | 1,2 g | 1,0 g | 0,7 g | 0,5 g | 0,2 g |
| 2,5-Diaminotoluol-sulfat | - | - | 0,2 g | 0,7 g | - | 0,7 g |
| 2-(2',5'-Diaminophenyl)-ethanol-sulfat | - | 0,3 g | 0,3 g | - | 1,0 g | 0,5 g |
| 6-Amino-2-methyl-3,4-dihydro-2H-1,4-benzoxazin-hydrochlorid | 0,2 g | - | 0,4 g | 0,4g | 0,2 g | 0,7 g |
| 2,4-Diamino-1-(2'-hydroxy-ethoxy)benzol-dihydrochlorid | 0,5 g | - | - | 0,7 g | 0,5 g | 0,3 g |

**Tabelle1** (Fortsetzung)

| **Beispiel** | **1** | **2** | **3** | **4** | **5** | **6** |
|---|---|---|---|---|---|---|
| **Vorstufe** | | | | | | |
| 2-Amino-4-[(2'-hydroxyethyl)-amino]anisol-sulfat | - | 0,3 g | - | - | - | - |
| 3-Aminophenol | - | - | 0,1 g | - | - | - |
| 5-Amino-2-methylphenol | 0,4 g | 0,3 g | 0,1 g | - | 0,4 g | - |
| Resorcin | - | 0,3 g | - | 0,1 g | - | 0,3 g |
| 2-Methylresorcin | - | - | 0,3 g | - | - | - |
| **Farbe auf gebleichtem Haar** | kastanie | rehbraun | dunkelbraun | dunkelbraun | dunkelbraun | dunkelbraun |
| **Reflex** | ziegelrot | karminrot | rotviolett | blauviolett | aubergine | stahlblau |

**Beispiel 7:** Haarfärbemittel in Cremeform

| | |
|---|---|
| 0,7 g | 4,5-Diamino-1-(2'-hydroxyethyl)-1H-pyrazol-sulfat |
| 1,2 g | 4-Amino-2-aminomethyl-phenol-hydrochlorid |
| 0,2 g | 6-Amino-2-methyl-3,4-dihydro-2H-1,4-benzoxazin-hydrochlorid |
| 0,2 g | Resorcin |

(fortgesetzt)

| | |
|---|---|
| 0,3 g | m-Aminophenol |
| 0,4 g | 5-Amino-2-methyl-phenol |
| 0,1 g | 2-Amino-6-chlor-4-nitrophenol |
| 15,0 g | Cetylalkohol |
| 3,5 g | Natrium-laurylalkohol-diglykolethersulfat (28prozentige wäßrige Lösung) |
| 3,0 g | Ammoniak (25prozentige wäßrige Lösung) |
| 0,3 g | Natriumsulfit, wasserfrei |
| 75,1 g | Wasser |
| 100,0 g | |

10 g der vorstehenden Farbträgermasse werden kurz vor Gebrauch mit 10 ml Wasserstoffperoxidlösung (6prozentige wäßrige Lösung) vermischt. Anschließend trägt man die gebrauchsfertige Mischung auf blonde Naturhaare auf und läßt sie 30 Minuten lang bei 40 Grad Celsius einwirken. Danach wird das Haar mit Wasser gespült und getrocknet. Das Haar hat eine kastanienbraun Färbung mit kupferrotem Reflex erhalten.

**Beispiel 8:** Haarfärbemittel in Gelform

| | |
|---|---|
| 0,5 g | 4,5-Diamino-1-(2'-hydroxyethyl)-1H-pyrazol-sulfat |
| 1,0 g | 2,5-Diaminophenylethanol-sulfat |
| 0,6 g | 6-Amino-3,4-dihydro-2H-1,4-benzoxazin-hydrochlorid |
| 0,4 g | Resorcin |
| 0,1 g | 2-Chlor-6-ethylamino-4-nitrophenol |
| 0,4 g | Natriumhydroxid, fest |
| 0,5 g | Ascorbinsäure |
| 7,0 g | Isopropanol |
| 3,0 g | Glycerin |
| 15,0 g | Ölsäure |
| 10,0 g | Ammoniak (25prozentige wäßrige Lösung) |
| 61,5 g | Wasser |
| 100,0 g | |

Man vermischt kurz vor dem Gebrauch 50 g der vorstehend beschriebenen Haarfarbträgermasse mit 50 ml Wasserstoffperoxidlösung (6prozentige wäßrige Lösung) und läßt das Gemisch 30 Minuten lang auf blonde menschliche Haare einwirken. Danach wird mit Wasser gespült und sodann getrocknet. Man erhält eine intensiv braune Färbung mit einem Kupferreflex.

**Beispiel 9:** Haarfärbelösung mit einem sauren pH-Wert

| | |
|---|---|
| 1,2 g | 4,5-Diamino-1-(2'-hydroxyethyl)-1 H-pyrazol-sulfat |
| 0,1 g | 6-Amino-2-methyl-3,4-dihydro-2H-1,4-benzoxazin-hydrochlorid |
| 0,4 g | 5-Amino-2-methylphenol |
| 0,2 g | 2-Methylresorcin |
| 0,3 g | Ascorbinsäure |
| 0,3 g | Natriumlaurylethersulfat |
| 1,1 g | Ammoniak (25prozentige wäßrige Lösung) |
| 96,4 g | Wasser |
| 100,00 g | |

Der pH-Wert der Haarfärbelösung wird auf einen Wert von 6,8 eingestellt (wobei je nach Bedarf eine verdünnte Ammoniaklösung oder verdünnte Salzsäure verwendet wird).

Unmittelbar vor der Anwendung werden 20 Gramm der Haarfärbelösung mit 20 Gramm einer 6prozentigen wäßrigen Wasserstoffperoxidlösung (pH = 6,8) vermischt und das erhaltene gebrauchsfertige Oxidationshaarfärbemittel (pH = 6,8) auf gebleichte Haare aufgetragen. Nach einer Einwirkungszeit von 30 Minuten bei Raumtemperatur werden die Haare mit Wasser gespült und getrocknet.

Das so behandelte Haar ist in einem dunklen Ton mit Auberginereflex gefärbt.

Alle Prozentangaben stellen soweit nicht anders angegeben Gewichtsprozente dar.

**Patentansprüche**

1. Haarfarbträgermasse, **dadurch gekennzeichnet, daß** sie als Entwicklersubstanz mindestens ein Diaminopyrazol der allgemeinen Formel (I) und als Kupplersubstanz mindestens ein Benzoxazin der allgemeinen Formel (II) enthält,

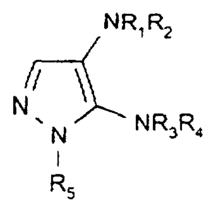

(I)

;

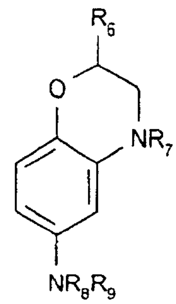

(II)

wobei in der allgemeinen Formel (1) die Substituenten R1 bis R5 unabgängig voneinander Wasserstoff, eine geradkettige oder verzweigte Alkylgruppe mit 1 bis 6 Kohlenstoffatomen, eine geradkettige oder verzweigte Hydroxyalkylgruppe mit 2 bis 4 Kohlenstoffatomen, eine geradkettige oder verzweigte Poly(hydroxy)alkylgruppe mit 3 bis 4 Kohlenstoffatomen oder einen unsubstituierten oder substituierten Benzylrest bedeuten, oder R1 und R2 sowie R3 und R4 jeweils ein heterocyclisches System bilden, oder R1 oder R2 mit R3 oder R4 verbrückt ist, R5 einen substituierten oder unsubstituierten Phenylrest bedeutet oder R5 mit R3 oder R4 verbrückt ist, während in der allgemeinen Formel (II) R6 gleich Wasserstoff oder einer verzweigten oder unverzweigten Alkylgruppe mit 1 bis 14 Kohlenstoffatomen, R7 gleich Wasserstoff, einer verzweigten oder unverzweigten Alkylgruppe mit 1 bis 6 Kohlenstoffatomen, einer geradkettigen oder verzweigten Hydroxyalkylgruppe mit 2 bis 4 Kohlenstoffatomen oder einem Benzylrest, und R8 und R9 unabhängig voneinander gleich einer geradkettigen oder verzweigten Alkylgruppe mit 1 bis 6 Kohlenstoffatomen, einer geradkettigen oder verzweigten Hydroxyalkylgruppe mit 2 bis 4 Kohlenstoffatomen oder einer geradkettigen oder verzweigten Poly(hydroxy)alkylgruppe mit 3 bis 4 Kohlenstoffatomen sind.

2. Haarfarbträgermasse gemäß Anspruch 1, **dadurch gekennzeichnet, daß** das Diaminopyrazol der Formel (I) ausgewählt ist aus Verbindungen mit R1 und R2 gleich Wasserstoff, R3 und R4 gleich Wasserstoff, einer Methylgruppe oder einer Hydroxyethylgruppe und R5 gleich einer Methylgruppe, einer Ethylgruppe, einer Isopropylgruppe, einer Hydroxyethylgruppe oder einer Benzylgruppe.

3. Haarfarbträgermasse gemäß Anspruch 1, **dadurch gekennzeichnet, daß** das Benzoxazin der allgemeinen Formel (II) ausgewählt ist aus Verbindungen der Formel (II) mit R6 gleich Wasserstoff oder einer Methylgruppe und R7, R8 und R9 gleich Wasserstoff.

4. Haarfarbträgermasse gemäß einem der Ansprüche 1 bis 3, **dadurch gekennzeichnet, daß** zusätzlich weitere Oxidationsfarbvorstufen aus der Gruppe bestehend aus p- Phenylendiaminderivaten oder m-Phenylendiaminderivaten und p- Phenylendiaminderivaten, m-Aminophenolen oder Resorcinen enthalten sind.

5. Haarfarbträgermasse gemäß einem der Ansprüche 1 bis 4, **dadurch gekennzeichnet, daß** die zusätzlichen weiteren Oxidationsfarbvorstufen ausgewählt sind aus der Gruppe bestehend aus 2,5-Diaminotoluol, 2-(2',5'-Diaminophenyl)-ethanol, 2-(2',5'-Diaminophenoxy)ethanol, 4-Aminophenol, 4-Amino-2-aminomethylphenol, 4-Amino-2-[(2'-hydroxyethyl)-aminomethyl]-phenol, 4-Amino-3-methylphenol, 2,4-Diamino-phenoxy-ethanol, 2,4-Diamino-5-fluortoluol, 2-Amino-4-(2'-hydroxyethyl)-aminoanisol, 2-Amino-4-chlor-6-methylphenol, 5-Amino-2-methyl-phenol, 4-Amino-2-hydroxyphenoxy-ethanol, 4-Amino-5-fluor-2-hydroxytoluol, 4-Amino-5-ethoxy-2-hydroxytoluol, Resorcin, 4-Chlorresorcin, 4,6-Dichlor-resorcin, 2-Methylresorcin, 4-Hydroxy-1,2-methylendioxybenzol, 4-(2'-Hydroxyethyl)amino-1,2-methylendioxybenzol, 4-Hydroxyindol, 3,5-Diamino-2,6-dimethoxypyridin oder deren Addukten mit organischen oder anorganischen Säuren sowie 1-Naphthol.

6. Haarfarbträgermasse gemäß einem der Ansprüche 1 bis 5, **dadurch gekennzeichnet, daß** zusätzlich direktziehende Farbstoffe enthalten sind.

7. Haarfarbträgermasse gemäß einem der Ansprüche 1 bis 6, **dadurch gekennzeichnet, daß** die Haarfarbstoffe in einem geeigneten kosmetischen Träger angewendet werden.

8. Haarfarbträgermasse gemäß einem der Ansprüche 1 bis 7, **dadurch gekennzeichnet, daß** die Gesamtkonzentration der Farbstoffe 0,1 bis 10 Gewichtsprozent beträgt.

9. Haarfarbträgermasse gemäß einem der Ansprüche 1 bis 8, **dadurch gekennzeichnet, daß** darin weitere übliche kosmetische Zusätze enthalten sind.

10. Haarfärbemittel, **dadurch gekennzeichnet, daß** es unmittelbar vor der Anwendung durch Vermischen einer Haarfarbträgermasse gemäß einem der Ansprüche 1 bis 9 mit einem Oxidationsmittel erhalten wird.

11. Haarfärbemittel nach Anspruch 10, **dadurch gekennzeichnet, daß** die Haarfarbträgermasse mit dem Oxidationsmittel in einem Verhältnis von 5:1 bis 1:3 vermischt wird.

12. Haarfärbemittel nach Anspruch 10 oder 11, **dadurch gekennzeichnet, daß** der pH-Wert des Haarfärbemittels gleich 3 bis 11 ist.

13. Verfahren zum Färben von Haaren, **dadurch gekennzeichnet, daß** ein Haarfärbemittel nach einem der Ansprüche 9 bis 12 auf das Haar aufgetragen wird, bei einer Temperatur von 15 bis 50°C 10 bis 45 Minuten lang einwirken gelassen wird, das Haar anschließend mit Wasser gespült wird, gegebenenfalls schamponiert wird und sodann getrocknet wird.

## Claims

1. Hair colour carrier mass **characterized in that** it comprises, as developer substance, at least one diaminopyrazole of the general formula (I) and, as coupler substance, at least one benzoxazine of the general formula (II),

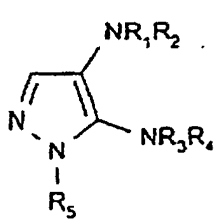

(I)

;

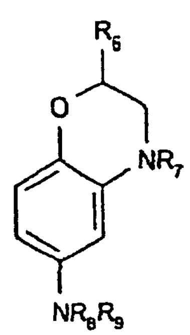

(II)

where, in the general formula (I), the substituents R1 to R5 are, independently of one another, hydrogen, a straight-chain or branched alkyl group having 1 to 6 carbon atoms, a straight-chain or branched hydroxyalkyl group having 2 to 4 carbon atoms, a straight-chain or branched poly(hydroxy)alkyl group having 3 to 4 carbon atoms or an unsubstituted or substituted benzyl radical, or R1 and R2, and R3 and R4 in each case form a heterocyclic system, or R1 or R2 is bridged with R3 or R4, R5 is a substituted or unsubstituted phenyl radical, or R5 is bridged with R3

or R4, while in the general formula (II) R6 is hydrogen or a branched or unbranched alkyl group having 1 to 14 carbon atoms, R7 is hydrogen, a branched or unbranched alkyl group having 1 to 6 carbon atoms, a straight-chain or branched hydroxyalkyl group having 2 to 4 carbon atoms or a benzyl radical, and R8 and R9, independently of one another, are a straight-chain or branched alkyl group having 1 to 6 carbon atoms, a straight-chain or branched hydroxyalkyl group having 2 to 4 carbon atoms or a straight-chain or branched poly(hydroxy)alkyl group having 3 to 4 carbon atoms.

2. Hair colour carrier mass according to Claim 1, **characterized in that** the diaminopyrazole of the formula (I) is chosen from compounds where R1 and R2 are hydrogen, R3 and R4 are hydrogen, a methyl group or a hydroxyethyl group and R5 is a methyl group, an ethyl group, an isopropyl group, a hydroxyethyl group or a benzyl group.

3. Hair colour carrier mass according to Claim 1, **characterized in that** the benzoxazine of the general formula (II) is chosen from compounds of the formula (II) where R6 is hydrogen or a methyl group and R7, R8 and R9 are hydrogen.

4. Hair colour carrier mass according to one of Claims 1 to 3, **characterized in that** further oxidation dye precursors from the group consisting of p-phenylenediamine derivatives or m-phenylene-diamine derivatives and p-phenylenediamine derivatives, m-aminophenols or resorcinols are additionally present.

5. Hair colour carrier mass according to one of Claims 1 to 4, **characterized in that** the additional further oxidation dye precursors are chosen from the group consisting of 2,5-diamino-toluene, 2-(2',5'-diaminophenyl)ethanol, 2-(2',5'-diaminophenoxy)ethanol, 4-aminophenol, 4-amino-2-aminomethylphenol, 4-amino-2-[(2'-hydroxyethyl)-aminomethyl]phenol, 4-amino-3-methylphenol, 2,4-diaminophenoxyethanol, 2,4-diamino-5-fluoro-toluene, 2-amino-4-(2'-hydroxyethyl)aminoanisole, 2-amino-4-chloro-6-methylphenol, 5-amino-2-methylphenol, 4-amino-2-hydroxyphenoxyethanol, 4-amino-5-fluoro-2-hydroxytoluene, 4-amino-5-ethoxy-2-hydroxytoluene, resorcinol, 4-chlororesorcinol, 4,6-dichlororesorcinol, 2-methylresorcinol, 4-hydroxy-1,2-methylenedioxybenzene, 4-(2'-hydroxyethyl)amino-1,2-methylenedioxybenzene, 4-hydroxyindole, 3,5-diamino-2,6-dimethoxypyridine or adducts thereof with organic or inorganic acids, and 1-naphthol.

6. Hair colour carrier mass according to one of Claims 1 to 5, **characterized in that** direct dyes are additionally present.

7. Hair colour carrier mass according to one of Claims 1 to 6, **characterized in that** the hair dyes are used in a suitable cosmetic carrier.

8. Hair colour carrier mass according to one of Claims 1 to 7, **characterized in that** the total concentration of the dyes is 0.1 to 10 percent by weight.

9. Hair colour carrier mass according to one of Claims 1 to 8, **characterized in that** further customary cosmetic additives are present therein.

10. Hair colorant **characterized in that** it is obtained directly prior to use by mixing a hair colour carrier mass according to one of Claims 1 to 9 with an oxidizing agent.

11. Hair colorant according to Claim 10, **characterized in that** the hair colour carrier mass is mixed with the oxidizing agent in a ratio of from 5:1 to 1:3.

12. Hair colorant according to Claim 10 or 11, **characterized in that** the pH of the hair colorant is 3 to 11.

13. Method of colouring hair, **characterized in that** a hair colorant according to one of Claims 9 to 12 is applied to the hair, left to act at a temperature of from 15 to 50°C for 10 to 45 minutes, the hair is then rinsed with water, is optionally shampooed and is then dried.

## Revendications

1. Masse support pour teinture pour cheveux, **caractérisée en ce qu'**elle contient comme substance de développement au moins un diaminopyrazole de formule générale (I) et comme substance de couplage au moins une benzoxazine de formule générale (II),

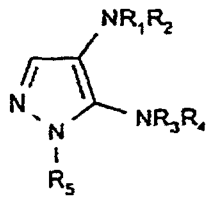

(I)

;

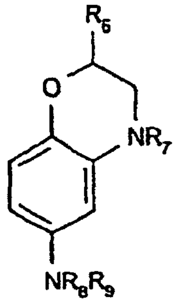

(II)

dans lesquelles, dans la formule générale (I), les substituants R1 à R5 représentent, indépendamment l'un de l'autre un hydrogène, un groupement alkyle linéaire ou ramifié comprenant 1 à 6 atomes de carbone, un groupement hydroxyalkyle linéaire ou ramifié comprenant 2 à 4 atomes de carbone, un groupement polyhydroxyalkyle linéaire ou ramifié comprenant 3 à 4 atomes de carbone ou un radical benzyle non substitué ou substitué, ou R1 et R2 ainsi que R3 et R4 forment à chaque fois un système hétérocyclique, ou R1 ou R2 forment un pont avec R3 ou R4, R5 signifie un radical phényle substitué ou non substitué ou R5 forme un pont avec R3 ou R4, alors que dans la formule générale (II), R6 représente un hydrogène ou un groupement alkyle ramifié ou linéaire comprenant 1 à 14 atomes de carbone, R7 représente un hydrogène, un groupement alkyle ramifié ou linéaire comprenant 1 à 6 atomes de carbone, un groupement hydroxyalkyle linéaire ou ramifié comprenant 2 à 4 atomes de carbone ou un radical benzyle et R8 et R9 représentent, indépendamment l'un de l'autre, un groupement alkyle linéaire ou ramifié comprenant 1 à 6 atomes de carbone, un groupement hydroxyalkyle linéaire ou ramifié comprenant 2 à 4 atomes de carbone ou un groupement polyhydroxyalkyle linéaire ou ramifié comprenant 3 à 4 atomes de carbone.

2. Masse support pour teinture pour cheveux selon la revendication 1, **caractérisée en ce que** le diaminopyrazole de formule (I) est choisi parmi les composés avec R1 et R2 représentant un hydrogène, R3 et R4 représentant un hydrogène, un groupement méthyle ou hydroxyéthyle et R5 représentant un groupement méthyle, éthyle, isopropyle, hydroxyéthyle ou benzyle.

3. Masse support pour teinture pour cheveux selon la revendication 1, **caractérisée en ce que** la benzoxazine de formule générale (II) est choisie parmi les composés de formule (II) avec R6 représentant un hydrogène ou un groupement méthyle et R7, R8 et R9 représentant un hydrogène.

4. Masse support pour teinture pour cheveux selon l'une quelconque des revendications 1 à 3, **caractérisée en ce qu'**elle contient en outre d'autres précurseurs de teintures d'oxydation du groupe constitué des dérivés de la p-phénylènediamine ou de la m-phénylènediamine et des dérivés de la p-phénylènediamine, des m-aminophénols ou des résorcines.

5. Masse support pour teinture pour cheveux selon l'une quelconque des revendications 1 à 4, **caractérisée en ce que** les autres précurseurs de teintures d'oxydation supplémentaires sont choisis parmi le groupe constitué par le 2,5-diaminotoluène, le 2-(2',5'-diaminophényl)éthanol, le 2-(2',5'-diaminophénoxy)éthanol, le 4-aminophénol, le 4-amino-2-amino-méthylphénol, le 4-amino-2-[(2'-hydroxyéthyl)-amino-méthyl]phénol, le 4-amino-3-méthylphénol, le 2,4-diaminophénoxyéthanol, le 2,4-diamino-5-fluorotoluène, le 2-amino-4-(2'-hydroxyéthyl)aminoanisole, le 2-amino-4-chloro-6-méthylphénol, le 5-amino-2-méthylphénol, le 4-amino-2-hydroxyphénoxyéthanol, le 4-amino-5-fluoro-2-hydroxytoluène, le 4-amino-5-éthoxy-2-hydroxytoluène, la résorcine, la 4-chlororésorcine, la 4,6-dichloro-résorcine, la 2-méthylrésorcine, le 4-hydroxy-1,2-méthylènedioxybenzène, le 4-(2'-hydroxyéthyl)amino-1,2-méthylènedioxybenzène, le 4-hydroxyindole, la 3,5-diamino-2,6-diméthoxypyridine ou leurs produits d'addition avec des acides organiques ou inorganiques ainsi que le 1-naphtol.

6. Masse support pour teinture pour cheveux selon l'une quelconque des revendications 1 à 5, **caractérisée en ce qu'**elle contient en outre des colorants directs.

7. Masse support pour teinture pour cheveux selon l'une quelconque des revendications 1 à 6, **caractérisée en ce que** les teintures pour cheveux sont utilisées dans un support cosmétique approprié.

8. Masse support pour teinture pour cheveux selon l'une quelconque des revendications 1 à 7, **caractérisée en ce que** la concentration totale en colorants est de 0,1 à 10% en poids.

9. Masse support pour teinture pour cheveux selon l'une quelconque des revendications 1 à 8, **caractérisée en ce qu'**elle contient d'autres additifs cosmétiques usuels.

10. Teinture pour cheveux, **caractérisée en ce qu'**elle est obtenue juste avant l'utilisation par mélange d'une masse support de teinture pour cheveux selon l'une quelconque des revendications 1 à 9 avec un oxydant.

11. Teinture pour cheveux selon la revendication 10, **caractérisée en ce que** la masse support pour teinture pour cheveux est mélangée avec l'oxydant dans un rapport de 5:1 à 1:3.

12. Teinture pour cheveux selon la revendication 10 ou 11, **caractérisée en ce que** le pH de la teinture pour cheveux est compris entre 3 et 11.

13. Procédé pour teindre des cheveux, **caractérisé en ce qu'**on applique une teinture pour cheveux selon l'une quelconque des revendications 9 à 12 sur les cheveux, on la laisse agir à une température de 15 à 50°C pendant 10 à 45 minutes, on rince ensuite les cheveux avec de l'eau, puis on shampouine le cas échéant, puis on sèche.